# EUROPÄISCHE PATENTSCHRIFT

(11) **EP 2 724 659 B1**
(45) Veröffentlichungstag und Bekanntmachung des Hinweises auf die Patenterteilung: **30.12.2015**
(21) Anmeldenummer: 13190290.0
(22) Anmeldetag: 25.10.2013
(51) Int. Cl.: A61B 1/00, A61B 1/12, B08B 9/032, A61B 19/00

(54) **Verfahren und Vorrichtung zum Reinigen und Desinfizieren von Endoskopen**
Method and device for cleaning and disinfection of endoscopes
Procédé et dispositif de nettoyage et de désinfection d'endoscopes

(30) Priorität: 25.10.2012 DE 102012020934
(43) Veröffentlichungstag der Anmeldung: 30.04.2014
(73) Patentinhaber: SciCan GmbH, 88299 Leutkirch (DE)
(72) Erfinder: Bärreiter, Robert Martin, 89335 Ichenhausen (DE)
(74) Vertreter: von Bülow, Tam

(56) Entgegenhaltungen:
- EP-A1- 0 711 529
- EP-A2- 1 779 769
- DE-A1- 10 208 035
- US-A1- 2007 100 204

## Beschreibung

Die Erfindung bezieht sich auf ein Verfahren und eine Vorrichtung zum Reinigen und Desinfizieren von Endoskopen gemäß dem Oberbegriff des Patentanspruches 1 bzw. 4.

Eine solche Vorrichtung ist aus der DE 103 21 991 B3 bekannt. Zur Validierung des Reinigungsergebnisses von Hohlkörpern ist jeder Hohlkörper über eine Kupplung mit einer Strömungsverengung und einem Drucksensor mit Speicherfunktion angeschlossen. Bei ausreichendem Durchfluss von Reinigungsflüssigkeit durch den Hohlkörper tritt an der Strömungsverengung ein Druckabfall auf. Bei ganz oder teilweise verstopftem Hohlkörper baut sich dagegen ein Staudruck auf, der von dem Drucksensor erfasst und dauerhaft gespeichert wird.

Die DE 102 08 035 B4 schlägt vor, jeden zu überprüfende Endoskopkanal mit einem Flüssigkeitsbehälter zu verbinden, der ein vorbestimmtes Volumen einer Flüssigkeit enthält. Eine gemeinsame Druckluftquelle ist an alle Flüssigkeitsbehälter angeschlossen und beaufschlagt die Behälter mit einem vorbestimmten Druck und treibt damit die Flüssigkeit durch den jeweiligen Endoskopkanal. Der Flüssigkeitspegel in den Behältern wird gemessen und eine Steuereinheit misst die Zeitdauer, innerhalb der das vorbestimmte Volumen aus den Flüssigkeitsbehältern durch den zu überprüfenden Endoskopkanal geflossen ist.

Die DE 603 06 345 T2 schlägt vor, alle Kanäle eines Endoskops an eine Druckkammer anzuschließen und separat über Ventile anzusteuern. Auch hier wird die Zeit gemessen, innerhalb der ein vorbestimmtes Volumen durch den individuellen Kanal geflossen ist. Ist der entsprechende Kanal abgekoppelt oder existiert ein Leck, so fließt die vorbestimmte Menge zu schnell aus der Kammer. Dadurch kann erkannt werden, dass der entsprechende Kanal nicht korrekt angeschlossen ist.

Die DE 10 236 565 C1 schließt jeden Arbeitskanal eines Endoskops an eine Quelle für unter Druck stehende Reinigungsflüssigkeit an. Ein Endoskoprohr ist unter Bildung eines Ringkanals in ein Reinigungsrohr eingesteckt. Der Ringkanal ist mit einer Quelle für unter Druck stehende Reinigungsflüssigkeit angeschlossen. Am distalen Ende des Reinigungsrohres ist eine Absaugpumpe angebracht, die dort einen Unterdruck erzeugt und Reinigungsflüssigkeit aus den Arbeitskanälen absaugt. Hierdurch wird die Druckdifferenz zwischen Eingang und Ausgang der Arbeitskanäle erhöht, ohne dass der Speisedruck unzulässig hohe Werte annimmt.

Die EP 1 433 412 A1 beschreibt ein Verfahren zum Erfassen eines Flusses in Endoskopkanälen. Jeder Endoskopkanal ist an einen Vorratsbehälter für Reinigungsflüssigkeit angeschlossen. Die Durchflussmenge der Flüssigkeit durch jeden Kanal wird gemessen durch Änderung des Volumens des Vorratsbehälters in Abhängigkeit von der Zeit.

Die EP 0 711 529 A1 und EP 0709056 A1 verwendet ein Durchflussprüfgerät für die Durchlässigkeit von Kanälen, die einzeln oder in Gruppen nacheinander geprüft werden. Bei nicht ausreichender Durchströmung eines Kanals wird das entsprechende Endoskop zur Aussonderung registriert.

In der Praxis kommt es öfter vor, dass einzelne Kanäle des zu reinigenden Endoskops nicht korrekt an die Zufuhr für Reinigungs- und Desinfektionsflüssigkeit oder Druckluft angeschlossen sind oder sich während der Reinigung lösen. Im praktischen Betrieb hat sich herausgestellt, dass eine eindeutige Erkennung, ob ein Endoskopkanal korrekt angeschlossen ist, aufgrund des Endoskopdesigns und der technischen Ausführungen der Kanalanschlüsse nur eingeschränkt und mit geringer Sicherheit detektierbar ist.

Die EP 1 779 769 A2 offenbart eine Vorrichtung und Verfahren gemäß dem Oberbegriff der Ansprüche 1 und 6.

Aufgabe der Erfindung ist es daher, Verfahren und Vorrichtung der eingangs genannten Art dahingehend zu verbessern, dass ein korrekter Anschluss von Endoskopkanälen an Zuleitungen für Reinigungsflüssigkeit, Desinfektionsflüssigkeit und/oder Druckluft einwandfrei erkannt werden kann. Als wünschenswerter Nebeneffekt soll auch die Reinigung und Desinfektion der Endoskopkanäle verbessert werden.

Diese Aufgabe wird durch die in den Patentansprüchen 1 bzw. 4 angegebenen Merkmale gelöst. Vorteilhafte Ausgestaltungen und Weiterbildungen der Erfindung sind den Unteransprüchen zu entnehmen.

Die Grundidee der Erfindung liegt darin, die einzelnen Endoskopkanäle gruppenweise mit unter Druck stehendem Fluid zu beaufschlagen und an den nicht mit Druck beaufschlagten Kanälen einer anderen Gruppe den Druck zu messen. Dabei stehen zumindest einige Kanäle der beiden Gruppen strömungsmäßig miteinander in Verbindung. Durch eine solche indirekte Messung an nicht direkt mit Druck beaufschlagten Kanälen kann der korrekte Anschluss der mit Druck beaufschlagten Kanäle einfach und mit hoher Zuverlässigkeit überprüft werden.

Durch die alternierende Umschaltung der Gruppen von Kanälen kann die Wirkung der Reinigungsflüssigkeit auf die Endoskopkanäle verstärkt werden, da sich die Flüssigkeit in den nicht angesteuerten Kanälen im Ruhezustand befindet. Durch den zyklischen Druckaufbau bei der alternierenden Kanalumschaltung wird die jeweils stehende Wassersäule immer wieder beschleunigt, was sich positiv auf die Reinigungswirkung auswirkt.

Das Verfahren nach der Erfindung enthält folgende Schritte:
- Alternierendes Zuführen von Fluid zu einer ersten Gruppe von Schläuchen und Absperren der Zufuhr zu einer zweiten Gruppe von Schläuchen,
- Messen des Druckes in mindestens einem Schlauch der Gruppe von abgesperrten Schläuchen,
- Vergleichen des gemessenen Druckes mit einem vorgegebenen Wert, und
- Erzeugen eines Fehlersignals, wenn der gemessene Druck von dem vorgegebenen Wert um einen vorgegebenen Betrag abweicht.

Generell wird bei der Reinigung von Endoskopen jeder Endoskopkanal mehrfach mit Reinigungsflüssigkeit und Desinfektionsflüssigkeit durchspült, mit voll entsalztem Wasser (VE-Wasser) ein- oder mehrfach nachgespült und anschliessend mit gereinigter oder aufbereiteter Druckluft durchgeblasen und getrocknet.

Wenn in der folgenden Beschreibung von Zufuhr von Fluid gesprochen wird, so bezieht sich dies nicht nur auf die Reinigungsflüssigkeit als solche sondern ebenfalls auf die Desinfektionsflüssigkeit, die Spülflüssigkeit und die Druckluft.

Im folgenden wird die Erfindung anhand eines Ausführungsbeispiels im Zusammenhang mit der Erfindung ausführlicher erläutert. Es zeigt:
- Fig. 1: Eine schematische Ansicht eines Endoskops mit Anschlüssen für die Reinigung; und
- Fig. 2: ein schematisches Flussdiagramm der Vorrichtung nach der Erfindung.

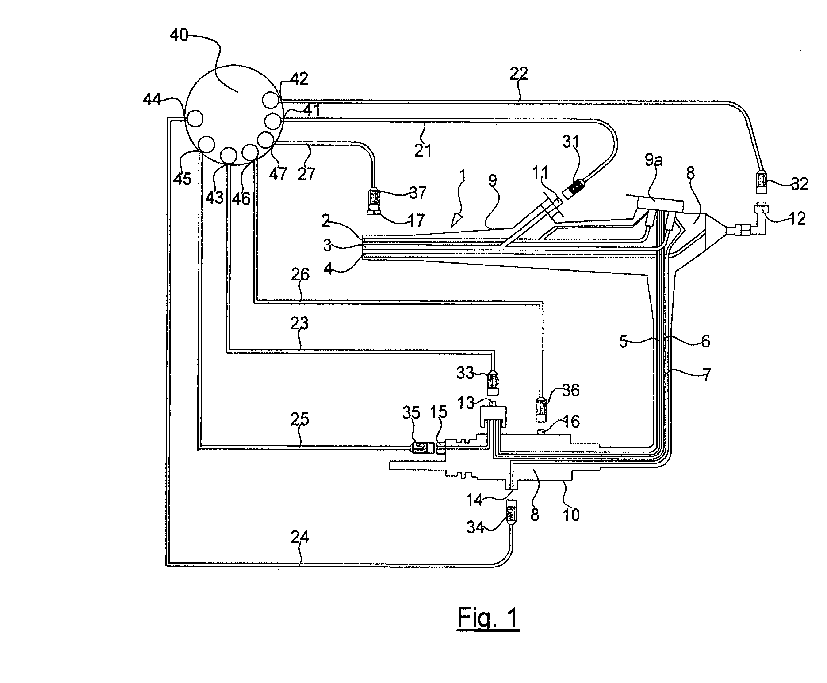

In Fig. 1 ist ein Endoskop 1 dargestellt, das eine Vielzahl von Kanälen 2, 3, 4, 5, 6 und 7 aufweist sowie einen flüssigkeitsdichten Hohlraum 8. Weiter hat das Endoskop mehrere Anschlüsse 11, 12, 13, 14, 15 und 16, über die Schläuche 21, 22, 23, 24, 25, 26 mit Anschlußstücken 31, 32, 33, 34, 35, 36 angeschlossen werden können. Alle Schläuche 20 bis 26 sind mit einem Adapter 40 einer Reinigungsmaschine verbunden, der eine Anzahl von Adapteranschlüssen 41, 42, 43, 44, 45, 46, 47 hat. Der Adapter befindet sich im Waschraum der Reinigungsmaschine und kann auch mehr als die dargestellten Adapteranschlüsse haben, um unterschiedliche Endoskope anschließen zu können. Nicht benötigte Adapteranschlüsse, wie zB der Adapteranschluß 47 werden dann beispielweise an dem Anschlußstück 37 durch einen Blindstopfen 17 verschlossen, wobei der Blindstopfen 17 eine kleine Öffnung haben kann, damit der Schlauch 27 bei einem Reinigungsvorgang durchspült wird.

Endoskope haben üblicherweise ein Bedienteil 9 und ein Versorgungsteil 10, wobei im Bedienteil 9 ein Kanaltrenner 9a vorgesehen ist. Der Hohlraum 8 ist sowohl im Bedienteil 9 als auch im Versorgungsteil 10 vorhanden und ist gegenüber den Kanälen 2 bis 7 abgedichtet. Einige der Kanäle 2, 3 und 4 des Bedienteiles 1 sind mit einigen der Kanäle 5, 6 und 7, die zum Versorgungsteil 8 führen, verbunden. Beispielsweise ist der Kanal 2 ein Wasserkanal, der durch den Kanaltrenner 9a mit dem Kanal 5 verbunden ist. Der Kanal 3 ist als Arbeitskanal und Absaugkanal vorgesehen und im Kanaltrenner 9a mit dem Kanal 7 verbunden. Der Kanal 4 ist hier der sog. Jetkanal, der bei dem dargestellten Endoskop nur im Bedienteil 9 verläuft und keine Verbindung zum Versorgungsteil 10 hat.

Generell ist anzumerken, dass für die Reinigung des Endoskops alle Kanäle mit Anschlüssen versehen sind, an die die Schläuche 21 bis 27 angeschlossen werden.

Es sei darauf hingewiesen, daß die Anzahl und Anordnung der Kanäle des Endoskops je nach Bauart und Verwendungszweck unterschiedlich ist und das hier dargestellte Beispiel nur exemplarisch zu verstehen ist.

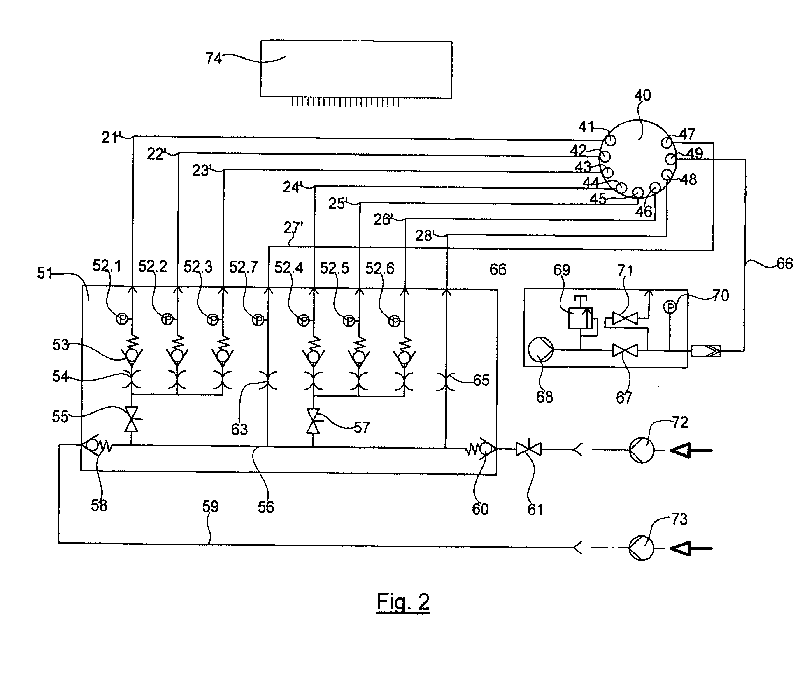

Fig. 2 zeigt die Vorrichtung nach der Erfindung mit dem Adapter 40, an den die Schläuche 21 bis 27 der Fig. 1 angeschlossen sind. In Fig. 2 hat der Adapter 40 neun Adapteranschlüsse 41 bis 49, von denen die Adapteranschlüsse 41 bis 47 mit den Schläuchen 21 bis 27 verbunden sind.

Im Ausführungsbeispiel der Fig. 2 sind die Adapteranschlüsse 41, 42 und 43 zu einer ersten Gruppe und die Adapteranschlüsse 44, 45 und 46 zu einer zweiten Gruppe zusammengefaßt. Die Adapteranschlüsse 41 bis 48 sind über Leitungen 21' bis 28' mit einer Verteilerbox 51 verbunden, wobei den Anschlüssen 41 bis 46 je ein Drucksensor 52.1 bis 52.6, ein Rückschlagventil 53 und eine Drosselstelle 54 zugeordnet ist. Die drei Adapteranschlüsse 41, 42 und 43 der ersten Gruppe sind gemeinsam über eine erste Absperreinrichtung 55 mit einer Zufuhrleitung 56 verbunden. Die Adapteranschlüsse 44, 45 und 46 der zweiten Gruppe sind in ähnlicher Weise über eine zweite Absperreinrichtung 57 mit der Zufuhrleitung 56 verbunden. Als Absperreinrichtungen sind hier elektrisch steuerbare Ventile 55, 57 gezeigt. Als Absperreinrichtungen können aber auch Pumpen verwendet werden, die im deaktivierten Zustand einen Durchfluss von Fluid unterbinden und somit ebenfalls eine Absperrfunktion haben. Beispielsweise können Schlauchquetschpumpen hierfür eingesetzt werden.

Die Zufuhrleitung 56 ist über ein Rückschlagventil 58 mit einer Flüssigkeitsleitung 59 verbunden, über die Reinigungs- oder Spülflüssigkeit zugeführt werden kann. Weiter ist die Zufuhrleitung 56 über ein Rückschlagventil 60 und ein drittes Ventil 61 mit einer Druckluftleitung verbunden. Die Rückschlagventile 53 und 58 sind so geschaltet, dass unter Druck stehendes Fluid von der Flüssigkeitsleitung 59 zu den jeweiligen Adapteranschlüssen 41 bis 46 gelangen kann. Ebenso ist das Rückschlagventil 60 so geschaltet, dass Druckluft von einer Druckluftquelle 72 zur Zufuhrleitung 56 und damit ebenfalls zu den Adapteranschlüssen 41 bis 46 gelangen kann.

Die Flüssigkeitsleitung 59 ist normalerweise mit dem Waschraum einer Reinigungsmaschine verbunden und erhält über eine Umwälzpumpe 73 die entsprechende Flüssigkeit des Waschraumes.

An die Zufuhrleitung 56 ist über eine Drosselstelle 63 eine Referenzleitung 27' mit einem Adapteranschluß 47 verbunden, wobei auch an diese Leitung ein Drucksensor 64 angeschlossen ist. Schließlich ist an die Zufuhrleitung 56 über eine Drosselstelle 65 eine weitere Leitung 28' angeschlossen, die mit dem Adapteranschluß 48 verbunden ist.

Zur Dichtigkeitsprüfung des Hohlraumes 8 des Endoskops ist an den Adapteranschluß 49 eine weitere Druckluftleitung 66 angeschlossen, die über ein viertes Ventil 67 mit einer Druckluftquelle 68 verbunden ist. An diese Leitung ist ein Druckregler 69 angeschlossen sowie ein Drucksensor 70 und ein viertes Ventil 71, das zur Entlüftung die Druckluftleitung mit Atmosphäre verbinden kann.

Alle genannten Ventile 55, 57, 61, 67 und 71 sind elektrisch steuerbar und mit einer elektronischen Steuereinrichtung 74 verbunden. Ebenso sind alle Drucksensoren und Pumpen mit der elektronischen Steuereinrichtung 74 verbunden.

Im folgenden wird die Funktionsweise der Vorrichtung der Fig. 2 beschrieben. Bei einem Reinigungsvorgang werden alle benötigten Schläuche mit den zugeordneten Anschlüssen des Endoskops verbunden, wobei die Schläuche und Anschlußstücke üblicherweise farblich gekennzeichnet sind.

Beim Reinigungsprozess werden die Ventile 55 und 57 alternierend geöffnet bzw. geschlossen. Ist das Ventil 55 geöffnet, so ist das Ventil 57 geschlossen und umgekehrt. In verschiedenen Reinigungs- und Spülvorgängen wird von der Umwälzumpe 73 über die Flüssigkeitleitung 59 und das Rückschlagventil 58 Reinigungs- oder Spülflüssigkeit zugeführt. Ist das Ventil 55 geöffnet und das Ventil 57 geschlossen, gelangt die Spülflüssigkeit von der Flüssigkeitsleitung 59 über die Drosselstellen 54 und die Rückschlagventile 53 zu den Adapteranschlüssen 41, 42 und 43 und durchströmt die zugeordneten Kanäle des Endoskops. Dadurch, dass einige dieser Kanäle auch mit den an die Adapteranschlüsse 44, 45 und 46 angeschlossenen Kanäle des Endoskops in Strömungsverbindung stehen, baut sich in den entsprechenden Kanälen und Leitungen ein Druck auf, der von den Drucksensoren 52.4 bis 52.6 der zweiten Kanalgruppe erfaßt wird, wobei deren Rückschlagventile 53 in Sperrrichtung mit Druck beaufschlagt sind. Ist eines der Anschlußstücke 31 bis 36 nicht korrekt angeschlossen oder der entsprechende Schlauch leck, so kann sich an den Drucksensoren 52 der zweiten Kanalgruppe kein entsprechender Druck aufbauen, was von den Drucksensoren 52 erfasst und von der elektronischen Steuereinrichtung 74 ausgewertet wird. Im Regelfall wird ein Alarmsignal erzeugt, das signalisiert, dass mindestens eines der Anschlußstücke nicht richtig befestigt ist.

Wird bei dem alternierenden Betrieb der Ventile 55 und 57 das Ventil 57 geöffnet und das Ventil 55 geschlossen, so wird der zweiten Kanalgruppe über die Adapteranschlüsse 44, 45 und 46 die Flüssigkeit zugeführt und der Staudruck von den zugeordneten Drucksensoren 52 der ersten Kanalgruppe erfasst.

Nach Beendigung der Reinigungs- und Spülvorgänge werden die Kanäle mit Druckluft über die Druckluftleitung 52 durchgeblasen. Diese gelangt von der Druckluftquelle 72 über das Rückschlagventil 60 zu der Zufuhrleitung 56 und ebenfalls im alternierenden Betrieb der Ventile 55 und 57 zu den entsprechenden Adapteranschlüssen und damit den entsprechenden Endoskopkanälen, so dass auch während des Vorganges des Ausblasens und Trocknens der jeweilige Druck der nicht aktivierten Kanalgruppe erfasst wird.

Die Dichtigkeit des Hohlraumes 8 des Endoskops wird von dem Drucksensor 70 überwacht. Ist dessen Adapteranschluss 36 nicht korrekt angeschlossen, so wird dies von dem Drucksensor 70 erfaßt.

Schließlich ist in an sich bekannter Weise noch ein Referenzkanal 27 (Fig. 1) und 27' (Fig. 2) vorgesehen, der die Druckverhältnisse der über die Flüssigkeitsleitung 59 und die Druckluftleitung 62 zugeführten Medien erfasst und mit den von der jeweils aktiven Kanalgruppe durch deren Drucksensoren 52 gemessenen Drücke vergleicht, wodurch Anomalien, wie z.B. verstopfte Kanäle, erfasst werden können. Der Referenzkanal ist im hier beschriebenen Ausführungsbeispiel primär durch den Schlauch 27 gebildet, der vorbestimmte Abmessungen hat, die einem fiktiven Endoskopkanal entsprechen und mit dem vom Drucksensor 52.7 gemessenen Druck einen Referenzwert liefert, der mit den von den übrigen Drucksensoren 52.1. bis 52.6 gemessenen Werten verglichen werden kann.

Wird beispielsweise die erste Kanalgruppe mit unter Druck stehender Flüssigkeit beaufschlagt, so ist das Ventil 55 geöffnet und das Ventil 57 geschlossen. Die Flüssigkeit gelangt unter anderem zu dem Adapteranschluss 41 und von dort über den Schlauch 21 zu dem Endoskopkanal 3 und gleichzeitig auch über die Leitung 7 in den Schlauch 24 und somit über den Adapteranschluss 44 zu dem Drucksensor 52.4. Da das zugeordnete Rückschlagventil 53 und das Ventil 57 geschlossen ist, baut sich an dem Drucksensor 52.4 ein Staudruck auf. Ist dieser gemessene Staudruck größer als ein vorbestimmter Wert, kann man sicher sein, daß der Adapteranschluss 31 korrekt angeschlossen ist. Ist umgekehrt bei dem alternierenden Betrieb der Ventile 55 und 57 das Ventil 57 geöffnet und das Ventil 55 geschlossen, so baut sich über die beschriebene Strömungsverbindung der Kanäle 7 und 2 der Staudruck an dem Drucksensor 52.1 auf.

In der elektronischen Steuerung 74 ist für jedes erfasste Endoskop die entsprechende Zuordnung der Kanäle und Drucksensoren gespeichert. So sind beispielsweise die Drucksensoren 52.1 und 52.4 einander zugeordnet. Je nach Endoskop können natürlich auch andere Zuordnungen vorliegen.

## Patentansprüche

1. Vorrichtung zum Reinigen und Desinfizieren von Endoskopen mit einem Adapter (40), an den mehrere Schläuche (21-26) angeschlossen sind, die mittels Anschlußstücken (31-36) an Kanäle eines Endoskops anschließbar sind, wobei jeder der Schläuche mit einer Zufuhrleitung (56) für ein Fluid verbindbar ist und jedem der Schläuche ein Drucksensor (52.1-52.6) zugeordnet ist, der den Druck in dem Schlauch misst und an eine elektronische Steuerung (74) meldet, **dadurch gekennzeichnet,**
**dass** eine erste Gruppe von mehreren Schläuchen (21, 22, 23) über eine erste Absperreinrichtung (55) mit der Zufuhrleitung verbunden ist,
**dass** eine zweite Gruppe von mehreren Schläuchen (24, 25, 26) über eine zweite Absperreinrichtung (57) mit der Zufuhrleitung (56) verbunden ist, wobei mindestens ein Schlauch der ersten Gruppe mit mindestens einem Schlauch der zweiten Gruppe verbunden ist,
**dass** die elektronische Steuerung (74) so ausgebildet ist, dass alternierend eine der Absperreinrichtungen geöffnet und die andere Absperreinrichtung geschlossen ist, und
**dass** die elektronische Steuerung (74) so ausgebildet ist, dass sie den von einem Drucksensor desjenigen Schlauches, dessen zugeordnetes Absperrventil geschlossen ist, gemessenen Druck mit einem vorgegebenen Druckwert vergleicht und ein Fehlersignal erzeugt, wenn der gemessene Druckwert und der vorgegebene Druckwert um einen vorgegebenen Differenzwert voneinander abweichen.

2. Vorrichtung nach Anspruch 1, **dadurch gekennzeichnet, dass** die Absperreinrichtungen Absperrventile (55, 57) sind.

3. Vorrichtung nach Anspruch 1, **dadurch gekennzeichnet, dass** die Absperreinrichtungen (55, 57) Pumpen, insbesondere Schlauchquetschpumpen sind, die im deaktiviertem Zustand einen Durchfluss von Fluid unterbinden.

4. Vorrichtung nach einem der Ansprüche 1 bis 3, **dadurch gekennzeichnet,**
**dass** in jedem der Schläuche (21-26) eine Drosselstelle (54) und ein Rückschlagventil (53) angeordnet ist und dass die Drucksensoren (52.1-52.6) zwischen dem Rückschlagventil (53) und dem Adapter (40) an den zugeordneten Schlauch (21-26) angeschlossen sind.

5. Vorrichtung nach einem der Ansprüche 1 bis 4, **dadurch gekennzeichnet,**
**dass** die Zufuhrleitung (56) über ein Rückschlagventil (58) mit einer Zufuhrleitung für Reinigungsflüssigkeit und/oder Desinfektionsflüssigkeit und über ein weiteres Rückschlagventil (60) und ein Absperrventil (61) mit einer Druckluftquelle (72) verbunden ist.

6. Verfahren zum Reinigen und Desinfizieren von Endoskopen, die mehrere an Schläuche anschließbare Kanäle aufweisen, **gekennzeichnet durch** folgende Schritte:
- alternierendes Zuführen eines Fluides zu einer ersten Gruppe von Schläuchen und Absperren der Zufuhr von Fluid zu einer zweiten Gruppe von Schläuchen, wobei mindestens ein Schlauch der ersten Gruppe mit mindestens einem Schlauch der zweiten Gruppe verbunden ist,
- Messen des Druckes in mindestens einem Schlauch der Gruppe von abgesperrten Schläuchen,
- Vergleichen des gemessenen Druckes mit einem vorgegebenen Wert und
- Erzeugen eines Fehlersignals, wenn der gemessene Druck von dem vorgegebenen Wert um einen vorgegebenen Betrag abweicht.

## Claims

1. Device for cleaning and disinfecting of endoscopes with an adapter (40) to which several tubes (21-26) are joined, which are joinable to channels of an endoscope via connectors (31-36), wherein each of the tubes is connectable with a supply pipe (56) for a fluid and each tube is allocated to a pressure sensor (52.1-52.6) that measures the pressure in the tube and reports it to an electronic control device (74), **characterized in that**
a first group of several tubes (21, 22, 23) is connected to the supply pipe via a first blocking installation (55),
**in that** a second group of several tubes (24, 25, 26) is connected to the supply pipe (56) via a second blocking installation (57), wherein at least one tube of the first group is connected with at least one tube of the second group,
**in that** the electronic control device (74) is provided **in that** alternatingly one of the blocking installations is opened and the other blocking installation is closed and
**in that** the electronic device (74) is provided **in that** it compares the measured pressure of a pressure sensor of that tube, which allocated blocking installation is closed, with a predefined pressure value and creates an error signal when the measured pressure value and the predefined value deviates by a predefined difference value.

2. Device according to claim 1, **characterized in that** the blocking installations are blocking valves (55, 57).

3. Device according to claim 1, **characterized in that** the blocking installations (55, 57) are pumps, in particular peristaltic pumps that prevent a flow of fluid in a deactivated state.

4. Device according to one of the claims 1 to 3, **characterized in that** a restriction (54) and a back pressure valve (53) are arranged in each of the tubes (21-26) and **in that** the pressure sensors (52.1-52.6.) between the back pressure valve (53) and the adapter (40) are joined to the allocated tube (21-26).

5. Device according to one of the claims 1 to 4, **characterized in that** the supply pipe is connected via a back pressure valve (58) with a supply pipe for cleaning fluid and/or disinfection fluid and via a further back pressure valve (60) and a blocking valve (61) with a compressed air source (72).

6. Method for cleaning and disinfecting endoscopes that comprise several channels connectable to tubes, **characterized by** the following steps:
- alternatively supplying of a fluid to a first group of tubes and blocking the supply of fluid to a second group of tubes, wherein at least one tube of the first group is connected with at least a tube of the second group,
- measuring the pressure in at least one tube of the group of blocked tubes,
- comparing the measured pressure with a predefined value and
- generating an error signal, if the measured pressure deviates for from the predefined value by a predefined amount.

## Revendications

1. Dispositif de nettoyage et de désinfection d'endoscopes avec un adaptateur (40), auquel plusieurs tuyaux (21-26) sont connectés, lesquels sont raccordables aux canaux d'un endoscope au moyen de pièces de raccordement (31-36), chacun des tuyaux étant relié à une conduite d'alimentation (56) de fluide et chacun des tuyaux étant associé à un capteur de pression (52.1-52.6) qui mesure la pression dans le tuyau et communique avec une commande électronique (74),
**caractérisé en ce que**,
un premier groupe de plusieurs tuyaux (21, 22, 23) est relié à la conduite d'alimentation par un premier dispositif d'arrêt (55),
un deuxième groupe de plusieurs tuyaux (24, 25, 26) est relié à la conduite d'alimentation (56) par un deuxième dispositif d'arrêt (57), dans lequel au moins un tuyau du premier groupe est relié avec au moins un tuyau du deuxième groupe,
la commande électronique (74) est conçue de manière à ce qu'alternativement un des dispositifs d'arrêt est ouvert et l'autre dispositif d'arrêt est fermé, et
la commande électronique (74) est conçue de manière à ce qu'elle compare une pression mesurée par un capteur de pression du tuyau dont la vanne d'arrêt associée est fermée, avec une valeur de pression prédéterminée et génère un signal d'erreur quand la valeur de pression mesurée et la valeur de pression prédéterminée s'écartent l'une de l'autre au-delà d'une valeur de différence prédéterminée.

2. Dispositif selon la revendication 1, **caractérisé en ce que** les dispositifs d'arrêt sont des vannes d'arrêt (55, 57).

3. Dispositif selon la revendication 1, **caractérisé en ce que** les dispositifs d'arrêt (55, 57) sont des pompes, en particulier des pompes péristaltiques, qui empêchent à l'état désactivé un écoulement de fluide.

4. Dispositif selon l'une quelconque des revendications 1 à 3, **caractérisé en ce que**,
dans chaque tuyau (21-26) est disposé un point d'étranglement (54) et un clapet anti-retour (53) et **en ce que** les capteurs de pression (52.1-52.6) sont connectés au tuyau (21-26) entre le clapet anti-retour (53) et l'adaptateur (40).

5. Dispositif selon l'une quelconque des revendications 1 à 4 **caractérisé en ce que**, la conduite d'alimentation (56) est reliée par un clapet anti-retour (58) à une conduite d'alimentation pour liquide de nettoyage et/ou un liquide de désinfection et par un autre clapet anti-retour (60) et une vanne d'arrêt (61) à une source d'air comprimé (72).

6. Procédé de nettoyage et de désinfection d'endoscopes, lequel présente plusieurs canaux raccordables sur des tuyaux, **caractérisé par** les étapes suivantes :
- alternativement délivrer un fluide à un premier groupe de tuyaux et fermer l'alimentation en fluide d'un deuxième groupe de tuyaux, au moins un tuyau du premier groupe étant relié avec au moins un tuyau du deuxième groupe,
- mesurer la pression dans au moins un tuyau du groupe des tuyaux fermés,
- comparer la pression mesurée avec une valeur prédéterminée et
- générer un signal d'erreur quand la pression mesurée s'écarte de la valeur prédéterminée au-delà d'une grandeur prédéterminée.
